# EUROPEAN PATENT APPLICATION

(11) **EP 1 393 672 A1**
(43) Date of publication of application: **03.03.2004**
(21) Application number: 03255023.8
(22) Date of filing: 13.08.2003
(51) Int. Cl.: A61B 5/0245, A61B 5/0428

(54) **Vital information measuring apparatus**

(30) Priority: 30.08.2002 JP 2002253239
(71) Applicant: Seiko Instruments Inc., Chiba-shi, Chiba (JP)
(72) Inventor: Yamasaki, Masaharu, Mihama-ku, Chiba-shi Chiba (JP)
(74) Representative: Sturt, Clifford Mark

(57) **Abstract**

Degradation of the efficiency of data transmission using a radio wave is to be suppressed. A communication circuit transmits heart beat information detected by electrodes to a master apparatus through an antenna using a radio wave. An inductor provided between the electrodes and an input section of the communication circuit suppresses input of the radio wave output by the antenna to the communication circuit through the electrode and waste of the same.

## Description

The present invention relates to a vital information measuring apparatus that detects vital information such as heart beat and transmits it using a radio wave.

Heart beat meters that are attached to a human body to measure the heart beat and that radio-transmits measured heart beat data through an antenna have been used as a kind of vital information measuring apparatus.

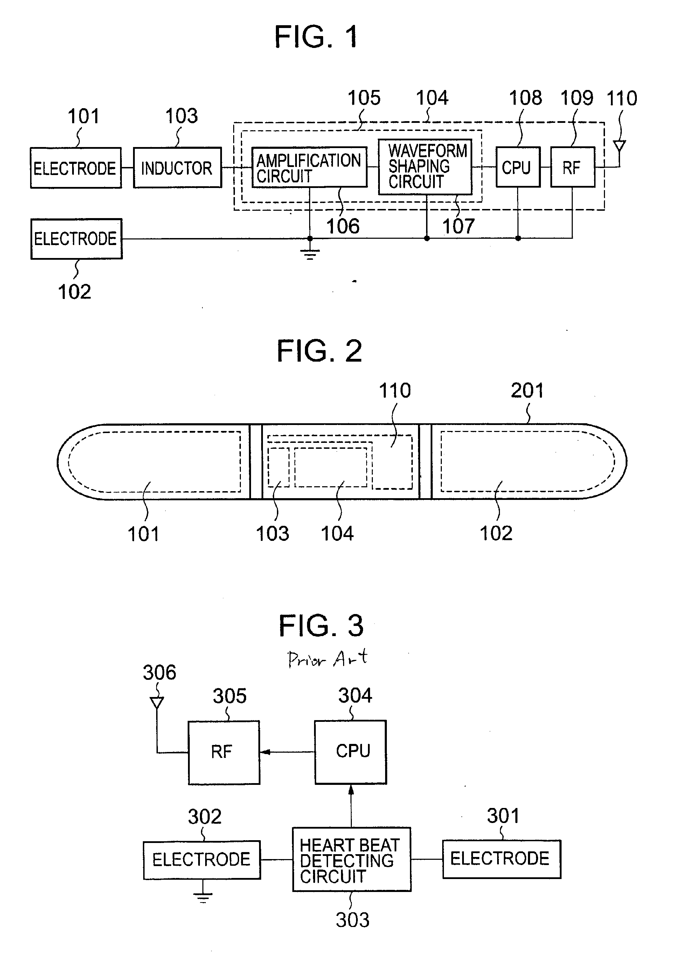

Fig. 3 is a block diagram of a conventional heart beat meter. The heart beat meter has a pair of electrodes for detecting heart beat (an electrode 301 on a positive side and an electrode 302 on a negative side (ground side)), a heart beat detecting circuit 303, a central processing unit (CPU) 304, a radio frequency (RF) circuit 305, and an antenna 306.

The electrode 302 is grounded, and the electrode 301 is an electrode for electrically detecting and fetching the heart beat of a human body. The heart beat detecting circuit 303 detects the heart beat of a user by detecting a voltage between the electrodes 301 and 302.

In the conventional heart beat meter, the heart beat detecting circuit 303 detects a signal of heart beat that is generated between the electrodes 301 and 302 attached to a human body and outputs it to the CPU 304. The CPU 304 outputs the heart beat signal to the radio frequency circuit 305 as heart beat data and controls the radio frequency circuit 305 such that the radio frequency circuit 305 transmits the heart beat data through the antenna 306.

Thus, the heart beat data of the human body is transmitted from the antenna 306 to a master apparatus (not shown) for performing various types of management such as display of the heart beat data.

However, since the electrodes 301 and 302 of the conventional heart beat meter are large-sized, a radio wave signal output by the antenna 306 is absorbed by the electrode 301, which results in a problem in that the efficiency of transmission using a radio wave is degraded.

Vital information measuring apparatus other than heart beat meters having a configuration including an antenna for transmitting data and an electrode for detecting vital information suffers from the problem of degradation of the efficiency of data transmission using a radio wave as described above.

It is an object of the present invention to suppress degradation of the efficiency of data transmission using a radio wave in a vital information measuring apparatus having vital information detecting means for detecting vital information, an antenna, and communication means for transmitting the vital information detected by the vital information detecting means using a radio wave through the antenna.

According to the present invention, there is provided a vital information measuring apparatus having vital information detecting means for detecting vital information, an antenna, and communication means for transmitting the vital information detected by the vital information detecting means using a radio wave through the antenna, characterized in that suppression means for suppressing input of the radio wave output by the antenna to the communication means through the vital information detecting means is provided between the vital information detecting means and an input section of the communication means. The suppression means disposed between the vital information detecting means and the communication means suppresses entry of a signal from the antenna into the communication means.

The suppression means may be an inductor or resistor.

Further, the inductor may be configured such that it has a self resonant frequency that is higher than the frequency of the radio wave that the communication means transmits through the antenna.

Further, the vital information detecting means may be configured as an electrode that is used by attaching it to a human body and that is for detecting the heart beat of the human body.

Embodiments of the invention will now be described by way of further example only and with reference to the accompanying drawings, in which:
Fig. 1 is a block diagram of a heart beat meter according to a mode for carrying out the present invention;
Fig. 2 is a front view of the heart beat meter according to the mode for carrying out the present invention; and
Fig. 3 is a block diagram of a conventional heart beat meter.

Fig. 1 is a block diagram of a vital information detecting apparatus according to a mode for carrying out the present invention, and it shows an example of a heart beat meter that is used by attaching it to the body of a user.

Fig. 2.is a front view of the vital information detecting apparatus shown in Fig. 1, parts identical to those in Fig. 1 being indicated by like reference numerals.

In Fig. 1 and Fig. 2, the heart beat meter has a pair of electrodes for detecting heart beat as the vital information detecting means (an electrode 101 on a positive side and an electrode 102 on a negative side (ground side)), an inductor 103 as the suppression means, a communication circuit 104 as the communication means, and an antenna 110.

The inductor 103, the communication circuit 104, and the antenna 110 are contained in a housing 201. The electrode 101 and the electrode 102 are mounted on the housing 201 such that they are exposed on the exterior of the housing 201 to allow them to be put in contact with a human body.

The communication circuit 104 has a heart beat detecting circuit 105, a central processing unit (CPU) 108, and a radio frequency (RF) circuit 109.

The electrodes 101 and 102 are used by attaching them to a human body and are electrodes for electrically detecting and fetching the heart beat of the human body that are formed in a large configuration ( e . g . , both of the electrodes 101 and 102 have a length of about 8 cm). The electrode 102 is grounded, and the electrode 101 is an electrode for electrically detecting and fetching the heart beat of a human body.

The heart beat detecting circuit 105 has an amplification circuit 106 for amplifying a heart beat signal input from the electrode 101 through the inductor 103 and a waveform shaping circuit 107 for shaping a waveform of a heart beat signal output by the amplification circuit 106. The heart beat detecting circuit 105 detects the heart beat of a user by detecting a voltage between the electrodes 101 and 102.

The inductor 103 is connected between the electrode 101 and an input section of the communication circuit 104. The inductance of the inductor 103 has a function of allowing a heart beat signal detected by the electrodes 101 and 102 to pass therethrough to the communication circuit 104 without any deterioration and suppressing a signal input from the antenna 110 through the electrode 101. Further, the inductor 103 has a self resonant frequency that is higher than the frequency of a radio wave that the communication circuit 104 transmits through the antenna 110.

Thus, the inductor 103 connected to the input section of the communication circuit 104 suppresses input of a signal in the frequency domain of a radio wave transmitted by the communication circuit 104 to the communication circuit 104 through the antenna 110. Since a circuit that can suppress input of the radio wave output by the antenna 110 to the communication circuit 104 through the electrode 101 is sufficient as the inductor 103, it is not essential to use the inductor 103, and a resistor may be used, for example. However, when matching of input impedance of the communication circuit 104 must be taken into consideration, an inductor is preferred to a resistor.

In the heart beat meter having a configuration as described above, the heart beat circuit 105 detects a signal of heart beat generated between the electrodes 101 and 102 attached to a human body and outputs it to the CPU 108 . The CPU 108 outputs the heart beat signal to the radio frequency circuit 109 as heart beat data and controls the radio frequency circuit 109 such that the radio frequency circuit 109 transmits the heart beat data through the antenna 110.

Thus, the heart beat data of the human body is transmitted from the antenna 110 using a radio wave to a master apparatus (not shown) for performing various types of management such as display of the heart beat data.

At this time, the radio wave signal output by the antenna 110 is received by the electrode 101 and is tempted to be input to the communication circuit 104, but the inductor 103 suppresses input of the signal from the antenna 110 receivedby the electrode 101 to the communication circuit 104. This makes it possible to suppress waste of the radio wave signal output from the antenna 110 in the communication circuit 104, and it is therefore possible to suppress degradation of the efficiency of data transmission from the heart beat meter using a radio wave.

As described above, the vital information measuring apparatus ( e . g . , a heart beat meter) according to the present mode for carrying out the invention is a vital information measuring apparatus having vital information detecting means (e.g., a pair of electrodes 101 and 102) for detecting vital information (e.g., heart beat information), an antenna 110, and a communication circuit 104 for transmitting the vital information detected by the vital information detecting means using a radio wave through the antenna 110, characterized in that suppression means (e.g., an inductor 103 or a resistor) for suppressing input of the radio wave output by the antenna 110 to the communication circuit 104 through the vital information detecting means is provided between the vital information detecting means and an input section of the communication circuit 104. It is thereforepossible to suppress degradation of the efficiency of data transmission when the vital information is transmitted from the communication circuit 104 through the antenna 110 using a radio wave.

While the present mode for carrying out the invention has been described using an example of a heart beat meter, it may be applied to vital information measuring apparatus for measuring various types of vital information such as blood pressure.

A vital information measuring apparatus according to the present invention makes it possible to suppress degradation of transmission efficiency when measured data is transmitted using a radio wave.

## Claims

1. A vital information measuring apparatus comprising:
vital information detecting means for detecting vital information ;
an antenna;
communication means for transmitting the vital information detected by the vital information detecting means using a radio wave through the antenna; and
suppression means for suppressing input of the radio wave output by the antenna to the communication means through the vital information detecting means,wherein the suppression means is provided between the vital information detecting means and an input section of the communication means.

2. A vital information measuring apparatus according to Claim 1, wherein the suppression means is an inductor.

3. A vital information measuring apparatus according to Claim 1, wherein the suppression means is a resistor.

4. A vital information measuring apparatus according to Claim 2, wherein the inductor has a self resonant frequency that is higher than the frequency of the radio wave that the communication means transmits through the antenna.

5. A vital information measuring apparatus according to Claim 1, wherein the vital information detecting means is an electrode that is used by attaching it to a human body and that is for detecting the heart beat of the human body.

6. A vital information measuring apparatus comprising:
a vital information detecting circuit for detecting vital information;
an antenna;
an transceiver for transmitting the vital information detected by the vital information detecting circuit using a radio wave through the antenna; and
a suppression circuit for suppressing input of the radio wave output by the antenna to the transceiver through the vital information detecting circuit, wherein the suppression circuit is provided between the vital information detecting circuit and an input section of the transceiver.
